# EUROPEAN PATENT SPECIFICATION

(11) **EP 3 519 832 B1**
(45) Date of publication and mention of the grant of the patent: **03.04.2024**
(21) Application number: 17800637.5
(22) Date of filing: 29.09.2017
(51) Int. Cl.: G01N 33/68

(54) **LABELED GLYCAN AMINO ACID COMPLEXES USEFUL IN LC-MS ANALYSIS AND METHODS OF MAKING THE SAME**
MARKIERTE GLYCANAMINOSÄUREKOMPLEXE FÜR DIE LC-MS-ANALYSE UND VERFAHREN ZUR HERSTELLUNG DAVON
COMPLEXES DE GLYCANE ET D'ACIDES AMINÉS MARQUÉS UTILES DANS L'ANALYSE EN LC-SM ET PROCÉDÉS POUR LES PRÉPARER

(30) Priority: 03.10.2016 US 201662403510 P
(43) Date of publication of application: 07.08.2019
(73) Proprietor: Waters Technologies Corporation, Milford, MA 01757 (US)
(72) Inventor: RAINVILLE, Paul, Princeton, MA 01514 (US); LAUBER, Matthew, A., North Smithfield, RI 02896 (US); GETHINGS, Lee, Egerton (GB); PLUMB, Robert, S., Milford, MA 01757 (US); COSGRAVE, Eoin, Mill Creek, WA 98012 (US); BROUSMICHE, Darryl, W., Grafton, MA 01519 (US)
(74) Representative: Forresters IP LLP
(86) International application number: PCT/US2017/054389
(87) International publication number: WO 2018/067398

(56) References cited:
- WO-A1-2016/069764
- WO-A1-2016/077548
- US-A1- 2007 269 895
- JENSEN PIA H ET AL: "Mucin-type O-glycosylation--putting the pieces together", FEBS JOURNAL, WILEY-BLACKWELL PUBLISHING LTD, GB, vol. 277, no. 1, 1 January 2010 (2010-01-01), pages 81-94, XP002598691, ISSN: 1742-464X, DOI: 10.1111/J.1742-4658.2009.07429.X [retrieved on 2009-11-17]
- MARCUS HOFFMANN ET AL: "Site-specific O -Glycosylation Analysis of Human Blood Plasma Proteins", MOLECULAR & CELLULAR PROTEOMICS, vol. 15, no. 2, 23 November 2015 (2015-11-23), pages 624-641, XP055433152, US ISSN: 1535-9476, DOI: 10.1074/mcp.M115.053546
- MATTHEW A. LAUBER ET AL: "Rapid Preparation of Released N -Glycans for HILIC Analysis Using a Labeling Reagent that Facilitates Sensitive Fluorescence and ESI-MS Detection", ANALYTICAL CHEMISTRY, vol. 87, no. 10, 19 May 2015 (2015-05-19), pages 5401-5409, XP055214900, ISSN: 0003-2700, DOI: 10.1021/acs.analchem.5b00758
- M Kimzey ET AL: "Development of an Instant Glycan Labeling Dye for High Throughput Analysis by Mass Spectrometry | Request PDF", Glycobiology, Annual Meeting of the Society-for-Glycobiology on Glycobiology -, Volume: 25, 1 November 2015 (2015-11-01), pages 1-4, XP055691648, Retrieved from the Internet: URL:https://www.researchgate.net/publicati on/296096139_Development_of_an_Instant_Gly can_Labeling_Dye_for_High_Throughput_Analy sis_by_Mass_Spectrometry [retrieved on 2020-05-05]
- Michael Kimzey ET AL: "Development of an Instant Glycan Labeling Dye for High Throughput Analysis by Mass Spectrometry", , 1 November 2015 (2015-11-01), XP055691666, Retrieved from the Internet: URL:https://www.europa-bioproducts.com/pro ductsheets/IPC_Glycan-Labeling-Dye-2015_ha ndout_v3_r2.pdf [retrieved on 2020-05-05]
- Kimzey Michael ET AL: "PROGRAM AND ABSTRACTS FOR 2015 ANNUAL MEETING OF THE SOCIETY FOR GLYCOBIOLOGY Glycobiology: Accelerating impact across the biomedical sciences ABSTRACT 190", PROGRAM AND ABSTRACTS FOR 2015 ANNUAL MEETING OF THE SOCIETY FOR GLYCOBIOLOGY. Glycobiology: Accelerating impact across the biomedical sciences, 1 December 2015 (2015-12-01), XP055777046, Retrieved from the Internet: URL:https://cyberleninka.org/article/n/449 631 [retrieved on 2021-02-17]
- YU YING-QING ET AL: "Enzyme-friendly, mass spectrometry-compatible surfactant for in-solution enzymatic digestion of proteins", ANALYTICAL CHEMISTRY, AMERICAN CHEMICAL SOCIETY, US, vol. 75, no. 21, 1 November 2003 (2003-11-01), pages 6023-6028, XP007906813, ISSN: 0003-2700, DOI: 10.1021/AC0346196

## Description

### BACKGROUND

Matrix-assisted laser desorption/ionization time-of-flight mass spectrometry ("MALDI-TOF-MS"), electrospray ionization ("ESI") and tandem mass spectrometry ("MS- MS") methods that utilize electrospray ionization with low-energy collision-induced dissociation ("CID") are each useful for the production of fragment ions to investigate the sequence and linkage of glycans and can play a crucial role in the characterization of glycosylation.

Peptide mapping is a method of selective fragmentation of drug substance or product into discrete peptides that can be used to confirm the desired product structure. Separation and identification of these fragments is performed in a reproducible manner so that in-depth analysis can identify minor even isobaric differences in protein primary structure such as errors in transcription of complementary DNA, point mutations, and post-translational modifications such as glycosylation, substitution and truncation. Due to the complexity and inherent variability of peptide mapping, a comparative procedure is performed where the peptide map of the test sample is compared to that of a reference substance prepared in a side-by-side experiment.

Ionization and mass spectrometry detection of O-linked glycans can be problematic, however, due to poor ionization efficiency of the glycan moieties. For example, MALDI-TOF-MS generates information relatively rapidly regarding the nature and diversity of glycans released from native, recombinant glycoproteins and other complex biological samples. Neutral glycans yield intense signals in the positive ion mode corresponding to sodium- cationized molecular species [M + Na]⁺ and are often accompanied by a weaker [M + K]⁺ion. Sialylated glycans, however, can be difficult to analyze using MALDI-TOF-MS and give a mixture of ions such as [M + Na]⁺, [M + K]⁺, [M-«H + (n + 1)Na]⁺ and [M-«H + (n + 1)K]⁺. Willy Morelle & Jean-Claude Michalski, Analysis of Protein Glycosylation by Mass Spectrometry, Nature Protocols 2, 1585-1602 (2007). Furthermore, sialylated glycans can lose a significant amount of sialic acid in the ion source or after the ion extraction from the ion source, considerably distorting the glycan profiles. To reduce this loss, sialylated glycans can be analyzed with time of flight ("TOF") instruments in the linear negative ion mode. Neutral glycans are not, however, detected in the negative ion mode. Id.

Proteomics research utilizing protein separation methods and mass spectrometry analysis has attracted considerable attention in recent years. A need exists, therefore, for robust detection of O-linked glycans with mass spectrometry alone or in combination with other detection techniques and to do so from a variety of sources including, but not limited to, glycan profiling experiments (glycomics) and glycopeptides, and in the analysis of glycolipids.
US 2007/269895 discloses a method for identifying and quantifying polyglycopeptides in a sample.Jensen, P.H. (2010). Mucin-type O-glycosylation-putting the pieces together. Febs Journal, 277(1), p. 81-84 discloses analytical approaches to characterising mucin-type O-glycans.
Hoffman, M. (2015). Site specific O-glycosylation analysis of human blood plasma proteins. Molecular & Cellular Proteomics, 15(2), p624-641 discloses site specific O-glycosylation of glycoproteins.

### SUMMARY

The invention is defined by the appended claims. Any embodiment not falling under the scope of the appended claims does not form part of the invention. Methods of characterizing protein glycosylation are provided herein. Advantages of the present methods include the elimination of laborious, irreproducible and problematic chemical hydrolysis of O-glycans (peeling) that then can require reductive amination to tag. The subject methods comprise the step of providing a plurality of glycosylated proteins or peptides. The proteins or the peptide can be denatured with a denaturing solution to produce a denatured mixture. The denaturing solution comprises a MS compatible surfactant, an organic solvent, urea or guanidine. The denatured mixture is combined with a plurality of proteases to produce a glycan amino acid complex. The glycan amino acid complex can be purified with HILIC SPE and isolated. In an embodiment of the methods described herein, a glycan can be attached to serine, threonine, tyrosine or hydroxylysine of the protein or the peptide. In an embodiment, the plurality of glycosylated proteins and/or the plurality of peptides are mixed with a plurality of proteases and a digestion buffer. In an embodiment, the digestion buffer is MS compatible. In an embodiment, the digestion buffer is not MS compatible. In an embodiment, the proteases are immobilized. In an embodiment, the proteases are not immobilized. In an embodiment, the proteases are a mixture of immobilized proteases and non-immobilized proteases. In an embodiment, the protease is mixed without other proteases. In an embodiment, the proteases are mixed in combination with other type of proteases. In an embodiment the proteases are sequentially mixed with a digestion buffer. In an embodiment, the denaturing solution comprises sodium 3-[(2-methyl-2-undecyl-1,3-dioxolan-4-yl)methoxy]-1-propanesulfonate. In an embodiment, immobilized endopeptidases and exopeptidases are concomitantly used to digest the peptide. In an embodiment, the peptide is digested using pepsin. In an embodiment, the peptide is digested with immobilized pronase. In an embodiment, the peptide is digested using trypsin. In an embodiment, the peptide is digested with immobilized trypsin. In an embodiment, the denaturing solution includes chymotrypsin and has a pH of 7.5 to 9.0. In an embodiment, the protein or peptide is denatured at a temperature between about 50°C to 90°C.

In an embodiment, the glycan amino acid complex is labeled with an amphipathic compound having a non-polar surface area of greater than about 200 Å and basic residue with a pKa greater than about 7 and a conjugate of the amphipathic compound and the glycan amino acid complex is formed. In an embodiment, non-polar surface area of the amphipathic compound is between about 200 Å and about 1000 Å. In an embodiment, the non-polar surface area of the amphipathic compound is between about 200 Å and about 500 Å. The glycan amino acid complex can be labeled with a rapid tagging reagent, or a reagent that does not rapidly label the glycan amino acid complex, to produce a labeled glycan amino acid complex. The labeled glycan amino acid complex is then detected and glycosylation of the protein or peptide is interpreted. In an embodiment, the protein or the peptide is characterized through a LC, MS or LC/MS analysis.

Further described are methods of making a labeled glycan amino acid complex comprising the steps of (a) releasing a glycan amino acid complex from the denatured protein or peptide and (b) labeling the glycan amino acid complex to produce analysis-ready glycan amino acid complexes. The methods of making a labeled glycan amino acid complex can also include the step of denaturing the protein or the peptide. In an embodiment, the peptides or the proteins are denatured with a denaturing solution to produce a denatured mixture. The denaturing solution can include a surfactant (MS compatible or not), an organic solvent, urea or guanidine. The methods of making the labeled glycan amino acid complex can also include a step of purifying the glycan amino acid complex and/or a step of purifying the tagged glycan amino acid complex. In an embodiment, the glycan amino acid complex is tagged with a rapid tagging reagent. In an embodiment, the plurality of proteases includes an enzyme or a plurality of different enzymes. In an embodiment, each different enzyme is added to the denatured mixture all at once, concomitantly, or sequentially.

### DETAILED DESCRIPTION

Glycan amino acid complexes and methodologies for making and analyzing the complexes are described herein. More specifically, a glycan, an O-linked glycan or an N-linked glycan, is conjugated to an asparagine, serine, threonine, tyrosine or hydroxylysine residue, respectively, to form a glycan amino acid complex. The glycan amino acid complex may comprise a single amino acid, or comprise a peptide of two to four covalently-linked amino acids. The glycan amino acid complex can be a complex having a short peptide combined with a glycan moiety. For example, in an embodiment, an O-linked glycan molecule attached to a peptide or a protein is cleaved, or otherwise released, from the protein or the peptide using one or more enzymes. The protein or peptide is digested, producing the glycan amino acid complex. Optionally, the glycan amino acid complex can be separated and/or purified from the other components present in a sample. Then, the glycan amino acid complex is tagged (labeled) with a derivatization reagent for detection and analysis of the glycan present in the sample.

More specifically and by way of example, a derivatization reagent can attach to the N-terminus of the amino acid of the O-linked glycan amino acid complex in a reagent solution, thus labeling the O-linked glycan amino acid complex to facilitate liquid chromatography-mass spectrometry ("LC-MS") analysis. Optionally, sample cleaning may be required to purify or isolate the O-linked glycan amino acid complex by solid phase extraction ("SPE") and/or separation by liquid chromatography prior to detection with a high resolution mass spectrometer or in nominal mass spectrometry.

In generating the glycan amino acid complex, one or more protease(s) that is referred to herein sometimes as a "cocktail" or a "proteases cocktail" in-solution are used such that the efficiency and/or specificity of labeling and the analysis are improved. In an embodiment, the O-linked glycan amino acid complexes are separated from a protein matrix via hydrophilic interaction chromatography solid phase extraction ("HILIC SPE") and injected onto a LC/MS system for analysis. The methodologies provided herein are designed to improve the efficiency of proteolysis in order to produce glycan amino acid complexes (i.e., O-linked glycan attached to the serine, threonine, tyrosine, or hydroxylysine) from a protein and/or peptide. These methodologies can also be used to analyze N-glycans.

The methodologies are useful in analyzing other saccharide-based protein modifications, including N-glycation, and glucan peptide/protein modifications. Glycation of proteins results from a non-enzymatic chemical reaction with reducing sugars and is a frequent product-related degradant in biopharmaceuticals, including recombinant monoclonal antibodies. Glycation often occurs by way of a covalent modification on lysine residues. Such an amino acid modification is also of relevance in diabetes-related diagnostics given that the glycation of a serum protein, such as hemoglobin, reports on average plasma glucose concentrations. As mentioned the present methodologies can also be used to analyze glucans, which are polysaccharides based glucose residues linked through glycosidic bonds. Given their therapeutic properties, some glucans are can be used as payloads in the creation of antibody conjugates

### Step 1 Protease Selection for Optimal Protein or Peptide Cleavage and Other Optional Preparatory Processing of Proteins and Peptides

Proteolytic digestion is a technique where a protein or peptide is enzymatically hydrolyzed into smaller peptides and/or amino acid residues. In-solution digestion of proteins is a technique where the protein/peptide is specifically dissolved and digested with proteolytic enzymes. Other techniques to cleave a protein or peptide employ enzymes immobilized on resins, or where enzymes and substrate co-adsorb onto a surface in order to produce the glycan amino acid complex.

In-solution digestion often leads to shortened preparation efforts and time. Notably, however, some proteins are not easily digested by proteases. Additional chemical processing steps can be required and used in combination or independently to produce the glycan amino acid complex. For example, a non-digestion approach, such as Edman degradation can be used to cleave the N-terminal amino acid residue from the protein or peptide. In this method, the amino terminal residue is labeled and cleaved from the peptide without disrupting the peptide bonds between other amino acid residues. Other O glycan-peptide digestion protocols involve immobilized proteases such as those described herein and in Slysz, Gordon W. et al., On-column Digestion of Proteins in Aqueous-Organic Solvents Rapid, Commun. Mass Spectrom 2003 : 17 1044-1050 (2003) at 1044-1050. Alternatively, other chemical cleavage techniques can be used including, but not limited the use of cyanogen bromide, 2 Nitro- thio-cyanobenzoic, O-iodozobenzoic acid, dilute acid or BNPS-skatole.

Enzymatic fragmentation of proteins, especially insoluble and proteolytic resistant proteins, however, can generate limited amounts of cleavage. For example, hydrophobic/proteolytic resistant proteins often do not yield a sufficient amount of peptides and can be under represented in the mixture submitted for analysis. See e.g., Russell, W. K. et al., Proteolysis in Mixed Organic-Aqueous Solvent Systems: Applications for Peptide Mass Mapping Using Mass Spectrometry, Anal. Chem. 2001, 73, 2682-2685. In short, proteolytic digestion and low amino acid coverage can affect an analysis. In particular, an inefficient proteolysis of a protein can comprise the ability to analyze a glycan amino acid complex. As such, the identification of the O-linked glycan attached to such proteins or peptides can be compromised.

To improve the protein digestion and as described herein, additives such as surfactants, organic solvents, and urea can be used to improve solubility and cleavage efficiency. Therefore, as a first step of the subject methods, in an embodiment, a protein or a peptide containing one or more O-linked glycan(s) can be denatured in a denaturing solution. In an embodiment, the denaturing solution comprises a mass spectrometry ("MS") compatible surfactant or otherwise referred to as a "cleavable detergent" or a "cleavable surfactant." Cleavable surfactants are rendered inactive by cleavage, often under acidic conditions, and in order to selectively remove the cleavage product. In the subject methods, the surfactant can improve in-solution protease digestion of protein and peptide in terms of speed and peptide coverage and not interfere with detection. Hence, the advantages of protein solubilization, rapid digestion, and high peptide (amino acid) coverages, are combined for mass spectrometry and liquid chromatography analyses. Sample preparation prior to mass spectrometry and liquid chromatography analysis can also include sample acidification.

As previously reported, improved in-solution tryptic digestion of proteins (in terms of speed and peptide coverage) can be achieved with the aid of a novel acid-labile anionic surfactant ("ALS, sodium 3-[(2-methyl-2-undecyl-1,3-dioxolan-4-yl)methoxy]-1-propanesulfonate "). Yu, Ying Qing, et al., Enzyme-Friendly, Mass Spectrometry-Compatible Surfactant for In-Solution Enzymatic Digestion of Proteins, Anal. Chem. 2003, 75 (21), 6023- 6028 at 6024. Unlike other molecules such as sodium dodecyl sulfate ("SDS"), ALS can solubilize proteins without inhibiting trypsin or other protease or common endopeptidases activity. In fact, trypsin activity has been evaluated in the presence of various denaturants, and there is little or no decrease in proteolytic activity in 0.1-1% ALS solutions (w/v). ALS can degrade rapidly at low-pH conditions and eliminates surfactant-caused interference. Acid-labile surfactants can enhance the rate and extent of in-solution protein digestion. *Id.* The acid-labile property of ALS can enable sample cleanup prior to MALDI MS or LC-MS analysis of protein digests, often without compromising the quality of the analysis.

ALS is marketed by Waters Corporation as the product RapiGest SF (also referred to sometimes as RapiGest surfactant) is an acid-cleavable anionic detergent marketed by Waters Corporation. RapiGest SF is one of many types of acid-labile anionic surfactants and a brandname for sodium 3-[(2-methyl-2-undecyl-1,3-dioxolan-4-yl)methoxy]-1-propanesulfonate. Other acid-labile surfactants are described in US 8,232,423. Other types of surfactants can be used in the present methodologies which are not acid labile include products such as Invitrosol (IVS), a homogeneous surfactant. Sodium deoxycholate ("SDC") is another MS-compatible detergent for in-solution digestion. SDC can enhance trypsin activity and can be removed by acidification without detrimental peptide loss. Another enzyme-friendly, mass spectrometry-compatible surfactant for in-solution enzymatic digestion of proteins is Protease MAX, the brandname for sodium 3-((1-(furan-2-yl)undecyloxy)carbonylamino)propane-1-sulfonate. This cleavable detergent is sensitive to heat and acid and is degraded during a typical trypsin digestion into the uncharged lipophilic compound 1-(furan-2-yl)undecan-1-ol and the zwitterionic 30 aminopropane-1-sulfonic acid (homotaurine), which can be removed by C18 solid phase extraction during sample work-up.

The effects of four commonly used surfactants, namely n-octyl glucoside ("OG"), Triton X-100 ("TX-100"), 3-[(3-cholamidopropyl)dimethylammonio]-1-propanesulfonate ("CHAPS") and sodium dodecyl sulfate ("SDS") have been studied for biological sample preparation on trypsin digestion and MALDI-MS of the resulting digest. *See,* Zhang, N. et al., supra. These surfactants were examined in detail within the context of using a two-layer method for MALDI matrix/sample preparation. *Id.* Non-ionic and mild surfactants, such as OG, TX-100 or CHAPS, were reportedly found to have no significant effect on trypsin digestion with surfactant concentrations up to 1%. *Id.*

In the present methodologies, the denaturating step can also include the addition of an organic solvent. As described by Russell WK, et al., "the rate of protein digestion imposes significant limitations on high-throughput protein identification using mass spectrometry. Proteins are readily digested by trypsin in the presence of organic solvents such as methanol, acetone, 2-propanol, and acetonitrile. The rates of protein digestion in organic solvents, as indicated by the abundances of digest fragment ions in the mass spectrum, are increased relative to aqueous solution. In addition, amino acid coverage for the analyzed proteins increases in the presence of the organic solvents, and proteins that are resistant to proteolysis are readily digested. Moreover, the tryptic digestion of a complex protein mixture in an organic-aqueous solvent system show significantly enhanced digestion for nearly all of the protein components. Enzymatic digestion in an organic-aqueous solvent system can be a rapid, simple, and effective peptide mass-mapping technique." *See* Russell WK, et al., Proteolysis in Mixed Organic-Aqueous Solvent Systems: Applications for Peptide Mass, Anal. Chem, 2001 (2682-2685) at 2682.

One approach to solubilizing proteins is to change the solvent system to increase the potential of accelerating proteolytic digestion by denaturing the protein. See e.g., Fink, W. et al., Characterization of the Unfolding of Ribonuclease A in Aqueous Methanol Solvents, Biochemistry 1987, 26 (6), pp. 1665-1671. Moreover, it has been reported that trypsin maintains its proteolytic activity in a variety of organic solvent systems, even at high concentration levels. See e.g., Welinder. K.G., Generation of Peptides Suitable for Sequence Analysis by Proteolytic Cleavage in Reversed-Phase High-Performance Liquid Chromatography Solvents, Anal. Biochem. 1988 174, 54-64 (where trypsin, chymotrypsin, elastase, lysyl endopeptidase (Achromobacter protease I), endoproteinase Arg-C (from mouse submaxillary gland), Staphylococcus aureus V8 protease, pepsin, and thermolysin in the presence of 20% methanol, ethanol, 2-propanol, and acetonitrile at 22 and 37 °C have been evaluated). The use of enzymes that retain high activity in organic or mixed solvents, combined with the denaturing and increasing solubility of substrate proteins in organic solvents, can significantly impact cleaving efficiency, amino acid coverage, and ultimately high-throughput analysis of proteins via in- solution digestion. As such, digesting proteins in mixed-solvent systems such as methanol-water, acetonitrile-water, 2-propanol-water, or acetone-water can be an effective means to producing glycan amino acid complexes.

A protein or peptide can also be denatured by elevating temperature. A thermal denaturation method compatible with MALDI-MS has been reported. See Park, Z.Y et al., Thermal Denaturation: A Useful Technique in Peptide Mass Mapping, Anal. Chem. Vol. 72 No. 11, 2000 2667-2670. In this method, unlike chemical denaturation, sample preparation steps are minimal and purification and concentration steps prior to MALDI mass analysis are reportedly not required. Thermal denaturation (50°C to 90°C) of a protein was shown to be accomplished in 20 min without the requirement of adding other denaturants. *Id.* Temperature can be used along with the previously noted denaturation techniques to the benefit of achieving a synergistic effect.

Applicable digestion techniques and protocols include, but are not limited to, digestions that use a single protease with a protocol identified below, or cocktail digestion that uses a combination of proteases and associated protocols. For example, as set out in Table 1, there are general guidelines for in-solution digestion by way of protease to protein ratio, temperature and time.

**TABLE 1**

| General Guidelines In-Solution Proteolytic Digestion | | | | |
|---|---|---|---|---|
| Protease | Ratio* A | °C | Buffer | Time** |
| Trypsin | 1:20 - 1:100 | 37 | 500 mM ammonium bicaronate pH 7-8 | 2-24 hours |
| Chymotrypsin | 1:20 - 1:100 | 37 | 500 mM Tris HCL pH 6 - 8 with 10 mM calcium chloride or 0.1 M ammonium bicaronate pH 8 with 10 mM calcium chloride | 2-24 hours |
| Asp N | 1:20 - 1:100 | 37 | 250 mM Tris HCL with 2.5 mM zinc acetate pH 6 | 2-24 hours |
| GluC | 1:20 - 1:100 | 37 | ammonium bicaronate pH 7-8 or 500 mM ammonium acetate pH 4 | 2-24 hours |
| Pepsin | 1:20 - 1:100 | 37 | 0.04 N HCL or 0.04 N or 20 mM sodium acetate pH 4.5 - 6TFA | 2-24 hours |
| Lys C | 1:20 - 1:100 | 37 | 500 mM ammonium bicarbonate pH 6 - 9 or 2M urea | 2-24 hours |

| | | | | |
|---|---|---|---|---|
| *protease to target protein **dependent on denaturation protocol | | | | |

Additional proteases useful in the present methods include Arg-C (clostripain), an endopeptidase that cleaves at the C-terminus of arginine residues, including the sites next to proline. Cleavage can also occur at lysine residues. This sequencing grade enzyme can be used alone or in combination with other proteases for protein analysis by mass spectrometry and other applications. Arg-C activity is optimal in the pH range of 7.6-7.9. In addition, as described below, pronase can be used to cleave the protein or peptide and generate the glycan amino acid complex.

To improve the efficiency, throughput and/or quality of proteolytic digestion, it would be advantageous to use immobilized proteolytic enzymes, including but not limited to the example techniques described in Slysz, Gordon W. et al., On-column Digestion of Proteins in Aqueous-Organic Solvents Rapid, Commun. Mass Spectrom: 17 (2003) at 1044-1050 and Ahn, Joomi et al, Pepsin Immobilized on High-Strength Hybrid Particles for Continuous Flow Online Digestion at 10, 000 psi, Anal Chem. 84 (2012) at 7256-7262. It has been shown that the immobilization of enzymes can minimize autolytic digestion and contamination of the sample with peptides and amino acids. In addition, with immobilization, a robust protease cocktail can be produced where structurally restraining the different enzymes will impede them from digesting one another. Efficient digestions could accordingly be established using an immobilized protease cocktail. In some embodiments, this technique utilizes enzymes that are immobilized to particles constructed of silica or organosilica, gels constructed of agarose of polyacrylamide, or membranes constructed of cellulose acetate, Nitrocellulose, and cellulose esters (CA, CN, and CE), polysulfone (PS), polyether sulfone(PES), polyacrilonitrile (PAN), polyamide, polyimide, polyethylene and polypropylene (PE and PP), polytetrafluoroethylene (PTFE), polyvinylidene fluoride (PVDF), polyvinylchloride (PVC).

In an embodiment, the protocol for digestion can include pepsin as a proteolytic enzyme. Pepsin can be used free (in solution) or immobilized. Pepsin can be supplied as a slurry in glycerol, sodium acetate and sodium azide as a preservative. To prepare the slurry for use, the slurry can be transferred to an antibody or resin preparation and washed or re-suspended in a digestion buffer (20 mM sodium acetate, pH 4.5). Proteins can be dissolved and/or suspended in the digestion buffer and added to the pepsin, incubated and mixed, followed by centrifuging to pellet the resin and collect the supernatant.

The proteolytic enzyme can also be trypsin or chymotrypsin. An embodiment, an immobilized chymotrypsin digestion protocol can include reaction conditions between pH 7.5 to 9.0 at 37°C. The reaction rate can be increased by increasing the enzyme to protein substrate ratio and the incubation temperature. For example, a typical digestion of 4 hours to overnight can be achieved using a ratio of approximately 1:25 enzyme to protein substrate. For accelerated chymotrypsin digestion (approximately 0.5 to 1.0 hour), 1: 10 enzyme to protein substrate is used. The digestion buffer can be a buffer such as 0.08 Tris-HCL, 0.1 M calcium chloride, pH 7.8 or another buffer such as 1.0 M Ammonium bicarbonate, 0.01 M calcium chloride, pH 8.0. Protein can be dissolved in the digestion buffer. Immobilized chymotrypsin can be washed with digestion buffer and the gel separated from the buffer after each wash by centrifugation. Immobilized chymotrypsin gel can be re-suspended and added to the sample. The enzyme substrate mixture can be incubated in a water bath for 2 to 18 hours at 37°C with rapid agitation. Immobilized chymotrypsin gel is separated from the digestion mixture as noted above.

### EXAMPLE I

### PROPHETIC TRPYSIN BASED PROTOCOL

To denature protein sample, add 50mM H4HCO3 or 50mM Tris-HCl (pH7.8), 1mM CaCh, until the guanidine HC1 or urea concentration is less than 1M. For digestion of native proteins, dissolve the protein in 50mM H4HCO3 or Tris-HCl buffer with a pH between 7 and 9. Trypsin is then added to a final protease: protein ratio of 1:20 (w/w). It is preferred that the protein or peptide concentration is at least 0.1mg/ml and incubate at 37°C for at least 4 hours overnight. This trypsin digest can be subjected to additional proteases or chemical digestion techniques in order to produce the glycan amino acid complex.

### EXAMPLE II

### PRONASE PROTOCOL

Glycoproteins (0.5-2 mg) were solubilized in 0.1% (w/v) RapiGest solution in 50 mM ammonium bicarbonate. Proteins were reduced with 10 mM dithiothretol and subsequently alkylated using 15 mM iodoacetamide. The pronase to protein mass was 1 :50 (w/w). Enzymatic digestion was performed at 37 °C overnight. In the case of AGP (alpha- 1 acid glycoprotein), the sialic acid residues were removed by addition of 1 unit of R2 (3,6,8,9)-neuraminidase after the pronase digestion was accomplished (the additional incubation time was 12 h). See, Yu, Ying Qing, et al., Identification of N-Linked Glycosylation Sites Using Glycoprotein Digestion with Pronase Prior to Tandem Time-of-Flight Mass Spectrometry, Anal. Chem. 2007, 79, 1731-1738.

### EXAMPLE III

### PROPHETIC IMMOBILZED PRONASE PROTOCOL

A prototype immobilized Pronase resin can be constructed by coupling Pronase enzymes (53702 EMD MILLIPORE) onto bridged ethylene hybrid (BEH) organosilica particles. Using triethoxysilyl butyraldehyde, proteins can be functionalized via their primary amines and reductive amination to exhibit silane functionality. Through a subsequent silane bonding, the protein can thereafter be bonded onto a resin or particle. The reaction mechanism can also be performed in reverse, wherein the particle is first bonded with the aldehyde moiety and a reductive amination reaction is used to couple proteins to the aldehyde. Moreover, the conjugation of the enzymes to the resin can also be accomplished by other chemical means, including but not limited to epoxide or carbodiimide based chemistry.

In brief, a solution containing 50-100 mg of Pronase can be prepared in 1-3 mL of 50 mM trisodium citrate at pH 5.0. One to two mL of solution containing 1 M sodium cyanoborohydride and 50-200 mg triethoxysilyl butyraldehyde is thereafter added to the Pronase solution and the reductive amination reaction is allowed to proceed at 10 to 50°C for 1 to 4 hours. 0.1 to 1.0 g of BEH particles are then added to the solution and the silane bonding is allowed to proceed for 0.5 to 12 hours. The mixture is then subjected to a salting out condition via the addition of 1 to 4 mL of 2 M sodium sulfate in 50 mM trisodium citrate at pH 5.0 and is rotated overnight at room temperature. Finally, the immobilization reaction is quenched by the addition of 0.5 to 2 mL of a basic solution, such as 1 M ethanolamine, and rotated for 2 hours at room temperature. Lastly, repeat washes of 50 mM trisodium citrate, and 1M sodium chloride are used to wash the Pronase immobilized BEH particles. See, Ahn, J. et al., Pepsin Immobilized on High-Strength Hybrid Particles for Continuous Flow Online Digestion at 10, 000 psi, Anal. Chem 84(16): 7256-7262.

Both off-line and online digestions can be performed with the immobilized Pronase BEH particles. For an online digestion, the methods described by Freije, J. Robert et al, Chemically Modified, Immobilized Trypsin Reactor with Improved Digestion Efficiency, J. Proteome Res., 2005, 4 (5), pages 1805-1813, can be applied. For an off-line digestion, immobilized Pronase can be packed into centrifugation-capable spin columns and used in an identical manner to immobilized trypsin spin columns, such as those made commercially available by Promega (Catalog V9012).

### Step 2 Producing the Glycan Amino Acid Complex

As noted above, the digestion step can involve mixing a plurality of proteases (referred to herein as a cocktail of proteases) together with buffer concentrations, to obtain appropriate pH and enzyme to protein ratios. The various proteases include, but not limited to combinations of trypsin, Lysyl endopeptidase ("Lys C"), pepsin, renin, chymotrypsin, elastase, carboxypeptidase, aminopeptidase, tripeptidase, N-aminopepsidase, glutamyl endopeptidase, peptidyl-ASP metallo-endopeptidase, clostripain and dipeptidase.

In the subject methodologies, upon digestion of the protein or peptide, the glycan amino acid complex is produced directly from the protein or peptide. As shown in Table 2 below, each protease, or enzyme, cleaves at different sites. For example, trypsin has specificity for the C-terminal side of Arg and Lys, and Chymotrypsin for the C-terminal side of hydrophobic residues (e.g, Leu, Met, Ala, and aromatics). Therefore, based on the cocktail, it is possible to fine-tune the cleavage in such a way so that glycans are cleaved together with their counterpart amino acid residues. The cocktail of proteases may also include certain chemicals that are known to digest proteins including, but not limited to, cyanogen bromide, 2 Nitro-5-thio-cyanobenzoic, O-Iodozobenzoic acid, dilute acid, BNPS-skatole.

**TABLE 2***

| Type | Agent | Specificity |
|---|---|---|
| Enzymatic | Trypsin (EC 3.4.21.4) | C-terminal side of Arg and Lys |
| | Chymotrypsin (EC 3.4.21.1) | C-terminal side of hydrophobic residues (e.g. Leu, Met, Ala, aromatics) |
| | Pepsin (EC 3.4.23.1 and 2) | Non-specific digest |
| | Lysyl endopeptidase (Lys-C endopeptidase) (EC 3.4.21.50) | C-terminal side of Lys |
| | Glutamyl endopeptidase (from S. aureus strain V8) (EC 3.4.21.19) | C-terminal side of Glu and Asp |
| | Peptidyl-Asp metallo-endopeptidase (endoprotein-ase Asp-N) | N-terminal side of Asp |
| | Clostripain (EC 3.4.22.8) | C-terminal side of Arg |
| Chemical | Cyanogen bromide | C-terminal side of Met |
| | 2-Nitro-5-thio-cyanobenzoic acid | N-terminal side of Cys |
| | O-Iodosobenzoic acid | C-terminal side of Trp and Tyr |
| | Dilute Acid | Asp and Pro |
| | BNPS-skatole | Trp |

| | | |
|---|---|---|
| *European Pharmacopoeta 5.0, 2.2.55, Peptide Mapping | | |

### Step 3 Purification or Isolation of the Glycan Amino Acid Complex

The glycan amino acid complex can be purified by utilizing hydrophilic interaction chromatography solid phase extraction ("HILIC SPE"). Protein samples are generally derived from complex matrices. Contaminants and non-protein materials present during the denaturation step can include DNA, RNA, and intact organelles. Such contaminants can impair detection of glycan amino acid complex and harm column efficiency as well as increase back pressure in the LC system. In addition, a complex sample matrix can contain compounds that interfere with downstream labeling reaction.

In an embodiment, to isolate and purify the glycan amino acid complex, a SepPak aminopropryl SPE sorbent in the form of a µElution plate can be conditioned with 200µL of water. The wells can be equilibrated with 200 µL of 15:85 water/acetonitrile. Acetonitrile diluted samples can be then loaded. The well can be washed with two (2) 600 µL volumes of 1:9:90 (v/v/v) formic acid/water/acetonitrile. The glycan amino acid complex can then be eluted with three (3) 30 µL volumes of SPE Elution Buffer (200 mM ammonium acetate in 5% ACN). Alternatively, a diol bonded or hydrophilic-lipophilic balanced sorbent (containing a sufficiently hydrophilic moiety) could be used for accomplishing this step.

### Step 4 Labeling of Glycan Amino Acid Complex

Different labeling techniques can be used to introduce a label to the glycan amino acid complexes that is capable of improving their detectability when an analyst employs either optical detection, namely fluorescence, or mass spectrometry.

In an embodiment, a rapid tagging reagent can be used to rapidly produce analysis ready O-linked glycan complexes or analysis ready N-linked glycan complexes. "Analysis ready O-linked glycan complexes" means O-linked glycans complexes that are ready for LC or MS analysis with minimal processing, or without any additional processing.

While not all groups have a proton affinity or charge tag group, useful steps include the labeling (also referred to as "tagging") of the glycan amino acid complexes with a labeling reagent (also referred to as a tagging reagent) comprised of a fluorescent moiety, a proton affinity/charge tag group(s), and an N-hydroxysuccinimide ester or carbamate reactive group as shown below:

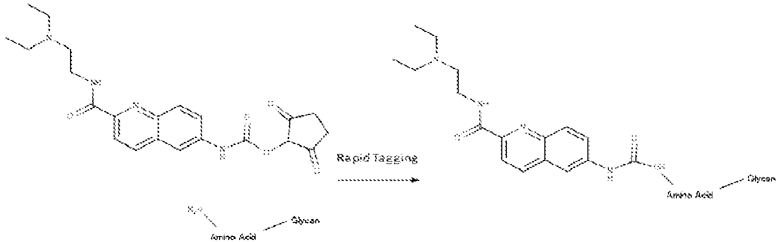

Exemplary reagents are shown in Table 3 below.

**TABLE 3**

| Labeling Reagent No. | Labeling Reagent Structure |
|---|---|
| Labeling Reagent-1 | 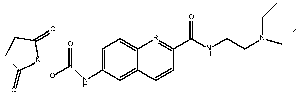 |
| Labeling Reagent-2 | 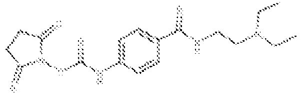 |
| Labeling Reagent-3 | 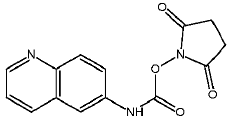 |
| Labeling Reagent-4 | 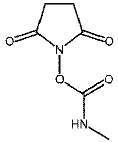 |
| Labeling Reagent-5 | 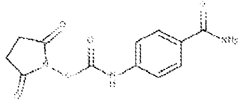 |
| Labeling Reagent-6 | 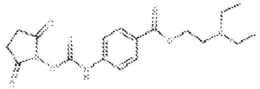 |

Other labeling reagents that can be used in connection with the methods described herein include those labeling reagents identified in US 2014/0350263 entitled Rapid Fluorescence Tagging of Glycans and Other Biomolecules with Enhanced MS Signals. See pages 2, lines 4 to page 4, line 9; page 11 line 4 to page 25, line 18 and page 29, line 1 to page 30 line 10. Additional labeling regents can also be found in US Pat. No. 7, 148,069 at Col. 8, 1. 56 to Col. 9, 1. 54 and Col. 15, 1. 22 to 29; US Pat No. 7,494,815 at Col. 7, 1. 19 to Col. 11, 1. 24; US Pat. No. 8, 124,792 at Col. 2, 1. 13 to Col. 4, 1. 5 and Col. 7, 1. 11 to Col. 17, 1. 20; and US Pat. No. 5,296,599 at Col. 4, 1. 66 to Col. 5, 1. 32 and Col. 5, 1. 66 to Col. 7, 1. 28.

Conditions of the labeling reaction, including temperature, organic solvent composition, organic solvent concentration, buffer composition, pH, ionic strength, molar excess of reagent, and time are selected and controlled such that desired reaction selectivity between primary amines and hydroxyl groups is achieved. In turn, the yield of labeled reaction product is optimized and the generation of "over-labeled" glycans (glycans or glycosylamines modified by >1 label) is minimized. In other words, the reaction product is optimized and the generation of over-labeled glycan amino acid complexes modified by any number of labels greater than the number of primary/secondary amines is minimized. However, sometimes over-labeling cannot be avoided.

The rapid tagging reagent facilitating the analysis can be synthesized based on rational design considerations which afford rapid labeling kinetics, high fluorescence quantum yield, and significantly enhanced MS detectability. The sample preparation can be dependent on reductive amination in addition to rapid tagging with NHS carbamate or HNS ester activated reagents. In this process, the glycan amino acid complex is reductively aminated in anhydrous conditions in order to minimize desialylation. The sample preparation transitions from aqueous to anhydrous conditions. In this reaction, a tagging reagent containing an aldehyde reacts in a condensation reaction with the amine of the glycan amino acid complex, resulting in an imine or Schiff base, which is reduced by a reducing agent to yield a secondary amine. The reaction is often performed in dimethyl sulfoxide containing acetic acid, but alternative approaches using tetrahydrofuran and methanol have been described. On the other hand, by utilizing the rapid tagging reagent, reductive amination can be eliminated and an aqueous reaction can be performed.

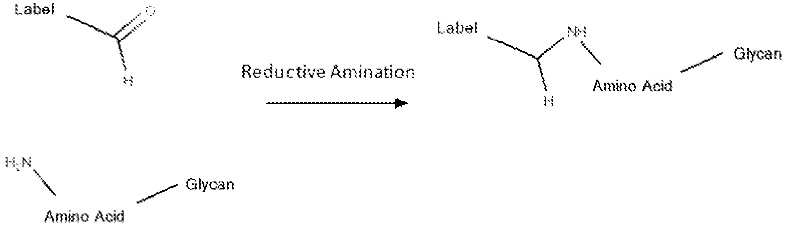

Rapid tagging of glycan amino acid complexes can be adopted in the laboratory using components of a GLYCOWORKS^{™} RAPIFLUOR-MS^{™} N-Glycan Kit. Likewise, these methods could be used to commercialize a new kit optimized specifically for the preparation of analysis ready, labeled glycan amino acid complexes. As provided herein, the optimized glycan sample preparation workflow for preparing an analysis ready, labeled glycan amino acid complex requires three steps: (1) producing the glycan amino acid complex from a protein; (2) labeling to impart a detectable chemical entity to the glycan amino acid complex; and (3) a clean-up step to remove potential interferences from the sample. The glycan amino acid complex can be rapidly reacted with one or more of the rapid tagging reagents and are thereby labeled with a tag comprised of an efficient fluorophore and a highly basic tertiary amine that yields enhanced sensitivity for both fluorescence and MS detection. A depiction of the structure of a rapid labeled glycan amino acid complex is shown immediately below.

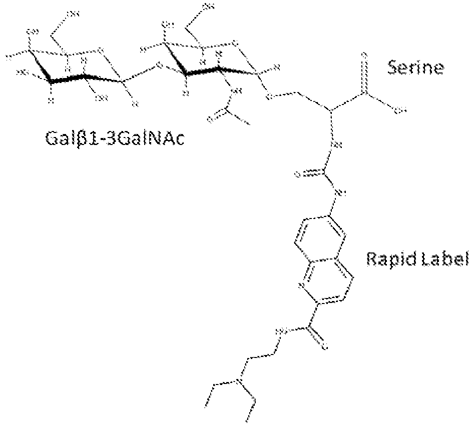

### Example of RapiFluor-MS Labeled Glycan Amino Acid Complex (Galβ1-3GalNAcαSer)

It is worth noting the rapid labeled glycan has a particularly unique linkage moiety, one that is distinct from the secondary amine linkage that comes from reductive amination reactions. Rapid labeled glycan amino acid complexes contain neutral (not acidic) urea linkages. This can impact the physicochemical characteristics and/or properties of the rapid labeled glycan, including their chromatographic retention and their fluorescence properties and other physiochemical properties such as isoelectric point ("pI"), acidity, basicity, hydrophobicity, hydrophilicity, ability to chelate metals, UV absorbance, fluorescence, absorbance in the visible spectrum, colorimetric changes, molecular size, affinity to interacting with binding partners (i.e. biotinylated residues to avidin or streptavidin, epitopes to paratopes), reduction/oxidation potential, propensity for crosslinking, cleavability (chemical and thermal), and polymeric substituents of varying length (i.e. poly ethylene glycol (PEG) 4 repeats, PEG 40 repeats).

As used herein, the marks GLYCOWORKS^{™} and RAPIFLUOR-MS^{™} are owned by applicant, Waters Technologies Corporation. The mark GLYCOWORKS^{™} is used in connection with sample preparation kits for laboratory use comprising biological standards, sample preparation devices, disposable cartridges, and chemical reagents for preparing samples for chromatography and mass spectrometry. Similarly, the mark RAPIFLUOR-MS^{™} is used in connection with chemical reagents for preparing samples for chromatography and mass spectrometry and in connection with sample preparation kits for laboratory use comprising biological standards, sample preparation devices, and disposable cartridges.

### Step 5 Purification or Isolation of the Labeled Glycan Amino Acid Complexes

In the present methodologies, prior to LC, MS, or LC-MS analysis and if required, the labeled glycan amino acid complexes may be subject to one of many purification type processing including but not limited to, dialysis, solid-phase extraction sample cleanup using reversed-phase (RP), hydrophilic interaction chromatography, or surfactant precipitation. Ying-Qing Yu, *supra.* Noteworthy, clean up methods can be time-consuming, in some instances, only partially efficient, and without the proper protocol, can lead to reduction in sample recovery. *Id*.

In an embodiment, to isolate and purify the labeled glycan amino acid complex from the labeling mixture, a SepPak aminopropryl SPE sorbent in the form of a µElution plate can be conditioned with 200µL of water. The wells can be equilibrated with 200 µL of 15:85 water/acetonitrile. Acetonitrile diluted samples can be then loaded. The well can be washed with two (2) 600 µL volumes of 1:9:90 (v/v/v) formic acid/water/acetonitrile. The glycan amino acid complex can then be eluted with three (3) 30 µL volumes of SPE Elution Buffer (200 mM ammonium acetate in 5% ACN). Alternatively, a diol bonded or hydrophilic-lipophilic balanced sorbent (containing a sufficiently hydrophilic moiety) could be used for accomplishing this step.

### Step 6 Glycosylation Analysis

The labeled glycan amino acid complexes described herein can be analyzed using various techniques, including but not limited to different types of separation and detection methods. The separation techniques can include, but are not limited to, hydrophilic interaction chromatography ("HILIC"), solid phase extraction ("SPE"), capillary electrophoresis, high pH anion exchange chromatography, or reversed phase liquid chromatography.

Furthermore, chromatographic devices such as those described in US Patent Pub. No.2013/03199086, provide multimodal chromatographic media (retention mechanism) and methods of analyzing glycans that provide high resolution between different biological macromolecules, unique selectivity based on size, composition, structure (e.g., isomerism, linkages) and/or charge. These types of columns allow the macromolecules eluted from the media to be detected by standard methodology (e.g., mass spectrometry, fluorescence detection) with no, or minimal, clean up or purification post-analysis and pre-detection (e.g., fluorescent tagging). Furthermore, to achieve sufficient sensitivity and selectivity for the complete separation of glycans, particularly N-linked glycans, chromatographic devices having high purity chromatographic materials ("HPCMs") comprising a chromatographic surface can be used. Here, the chromatographic surface (retention mechanism) has a hydrophobic surface group and one or more ionizable modifiers.

Detection methods can include chromatographic detection such as high pressure liquid chromatography ("HPLC"), ultra-high performance liquid chromatography (UHPLC), supercritical fluid chromatography, ultra violet ("UV") detection, fluorescence detection, matrix assisted laser desorption ionization mass spectrometry, electrospray ionization mass spectrometry, and/or pulsed amperometric detection

As described herein, characterization and monitoring of glycosylation of proteins and peptide are important in the detection of disease states and the manufacturing of biopharmaceuticals. Glycosylation profiles are most often assessed by means of released glycan analyses, wherein samples are often prepared by techniques that are notoriously time-consuming or lead to compromises in MS sensitivity. These shortcomings have been addressed by enabling unprecedented sensitivity for glycan detection while also improving the throughput of glycan sample preparation.

Equally important as the efficiency and sensitivity gains afforded by this new sample preparation approach, and the associated methodologies, is its robustness and its ability to produce results consistent with historical glycan profiling. Furthermore, glycan data generated from the glycan amino acid complexes can be used to interrogate glycan databases but first require conversion into a standardized value called a Glucose Unit ("GU") value. Hence, another benefit of implementing a calibration standard is the ability to convert existing glycan data into a format that makes exploration of glycan databases possible. The converted data can be used in a discovery process, where samples of unknown glycan composition are under investigation. Once the glycan is converted to GU values, users are able to interrogate online databases to gain insight into the potential glycan structures that may exist in their samples, potentially reducing the time required to perform a typical chromatography, calibration is performed frequently, sometimes as often as after every sample analysis.

Chromatographic analysis can also be performed directly on the obtained derivatization mixture and such analysis includes, but is not limited to, high pressure liquid chromatography ("HPLC"), ultrahigh performance liquid chromatography (UHPLC), mass spectrometry, supercritical fluid chromatography, ultraviolet ("UV") and/or fluorescent ("FLR") detection. Separation, however, is optionally including as described herein SPE offline and SPE online techniques.

To detect fluorescent ("FLR") labeled glycan amino acid complexes, hydrophilic interaction liquid chromatography ("HILIC") coupled with fluorescent detection can be used. For separation processing and in comparison to certain conventional high performance liquid chromatography ("HPLC") methods, a column packed with amide bonded bridged ethylene hybrid particles (herein after referred as "BEH glycan column" or "BEH column") operating in HILIC mode can provide improvements in peak resolution with the ability to separate both neutral and acidic labeled glycan amino acid complexes. The BEH glycan column enables and produces reproducible separation data and in less time spent for method optimization.

BEH columns utilize hybrid-silica BEH, bridged technology particles functionalized with a stable, amide-containing species. BEH technology has given rise to numerous particle size stationary phases ranging from 1.7 to 5 µm diameters to provide a bridge between HPLC and ultra-performance liquid chromatography ("UPLC") technology platforms. BEH particles offer peak shape and efficiency for basic analytes, a rational array of chromatographic selectivity and improvements in chemical stability at mobile phase extremes, particularly at elevated pH. The resolving power of the BEH glycan/BEH amide columns is due in part to porous particles with an optimal concentration of amide ligands for associated applications. The column can be optimized for use with either a UPLC or HPLC system.

In order to take full advantage of the BEH glycan column, or simply any HILIC-based profiling of labeled, glycan amino acid complexes, a dextran calibration ladder (hereinafter sometimes referred to as a "dextran ladder" or "dextran ladder standard") can be used. The glycan profile obtained from a HILIC/FLR system can be calibrated against the dextran ladder and assigned with glucose unit (GU) values. For example, one such known ladder, the 2AB-labeled dextran calibration ladder (Waters Corporation, part number 186006841), is different than other commercial offerings. The average molecular weight of the glucose homopolymer is higher (~4,500 Dalton), therefore, the "workable" GU value range is twice as much as other dextran ladder standards; the observed GU goes from 2 to 30. Hence, large glycan retention time assignment is improved. The purity and structural integrity of the dextran ladder can be assessed by both HILIC and mass spectrometry ("MS").

The elution times of a glycan (as represented by labeled, glycan amino acid complexes) can be expressed in glucose units ("GU") by reference to the dextran ladder. Each individual glycan structure has a GU value which is directly related to its linkages and constituent monosaccharides. The GU value can be used to predict structures because each monosaccharide in a specific linkage contributes specifically to the GU value of a given glycan. Therefore, the dextran ladders provided herein can be used to calibrate the LC runs against day-to-day or system-to-system changes. The GU value is calculated by fitting a fifth order polynomial distribution curve or cubic spline curve to the dextran ladder, then using this curve to allocate GU values from retention times. The GU values for glycans can be very reproducible, with standard deviations being less than 0.3 between columns. This allows direct comparison with database values collected from a range of instruments over a period of time.

Having GU values, databases with glycans stored in values of GU can be interrogated to aid in elucidating the potential glycan structures existing within a glycan population. A dextran ladder also provides a quality controlled standard that can be used to calibrate chromatograms obtained on different instruments in different labs. Retention times captured using HILIC- FLR instrumentation will vary from instrument to instrument and lab to lab. By converting retention times to GU values, the resulting data can be used to compare information between different locations both on-site and off-site. While the use of GU values is used as an example here, other chromatographic methods can benefit from the use of a dextran calibration, including but not limited to reversed phase chromatography and mixed mode iterations of both reversed phase and HILIC.

Rapid labeling of glycan amino acid complexes can simplify the preparation of glycan samples for analysis, particularly when applied to the analysis of O-glycosylation. Yet, it is not a trivial task to produce a dextran ladder that is appropriate for use with these types of labeled compounds, as would be needed to generate so-called glucose unit values. As described in PCT Application No. PCT/US2015/060326, published as WO 2016/077548, a two-step process for tagging of reducing glycans can provide for tuning of chromatographic response and chemical properties, such as fluorescence and MS activity and multiple tags with differing detection properties. Each step differs in the nature of attachment to the reducing glycan. The term "reducing glycan" means reducing sugar, reducing saccharide, reducing polysaccharide (hetero-saccharide different sugar units, or different monosaccharides, or homo-saccharide) and includes any aldehyde terminated saccharide such as chitotriose, chitobiose, galactose-β-(1-3)-GalNAc-Glycan, mannotriose-di-(N-acetyl-D-glucosamine) and maltrose. A reducing glycan or a reducing sugar is any sugar capable of acting as a reducing agent because it has a free aldehyde group. In relation to glycan amino acid complexes, it is likely that the above mentioned technique could be used to make a chemically similar dextran ladder. In one specific embodiment, dextran could be reductively aminated with serine and then derivatized with RapiFluor-MS to produce the structure depicted immediately below.

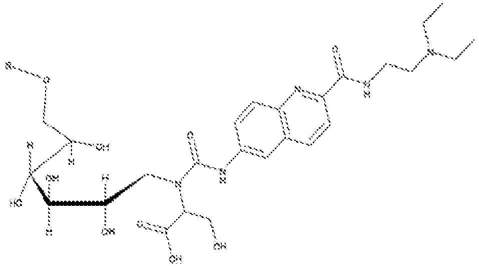

## Claims

1. A method of characterizing glycosylation of a protein or a peptide comprising the steps of:
providing a plurality of O-glycosylated proteins and/or peptides;
mixing the plurality of O-glycosylated proteins and/or peptides with a plurality of proteases and a digestion buffer, wherein the plurality of proteases cleaves the protein and/or the peptide with serine, threonine, tyrosine or hydroxylysine in an in-solution proteolytic digestion process to produce an O-glycan amino acid complex; and
tagging the O-glycan amino acid complex with a tagging reagent to produce a labeled O-glycan amino acid complex;
detecting the labeled O-glycan amino acid complex; and
characterizing glycosylation of the protein and/or the peptide through a LC, MS or LC/MS analysis through the detection of the labeled O-glycan amino acid complex.

2. The method of claim 1, wherein the digestion buffer is MS compatible.

3. The method of claim 1, wherein the protease is mixed alone or in combination with other type of proteases.

4. The method of claim 3, wherein the proteases are sequentially mixed with the digestion buffer.

5. The method of claim 1, wherein the protein is partially digested.

6. The method of claim 1, wherein the method further comprises the step of purifying the glycan amino acid complex with HILIC SPE and isolating the glycan amino acid complex from the matrix, or wherein the method further comprises the step of purifying the labeled glycan amino acid complex and isolating the labeled glycan amino acid complex.

## Patentansprüche

1. Verfahren zum Charakterisieren einer Glykosylierung eines Proteins oder eines Peptids, das die Schritte umfasst:
Bereitstellen einer Mehrzahl von O-glykosylierten Proteinen und/oder Peptiden;
Mischen der Mehrzahl von O-glykosylierten Proteinen und/oder Peptiden mit einer Mehrzahl von Proteasen und einem Verdauungspuffer, wobei die Mehrzahl von Proteasen das Protein und/oder das Peptid mit Serin, Threonin, Tyrosin oder Hydroxylysin in einem Prozess der proteolytischen Verdauung in Lösung spaltet, um einen O-Glykan-Aminosäure-Komplex zu bilden; und
Taggen des O-Glykan-Aminosäure-Komplexes mit einem Tagging-Reagens, um einen markierten O-Glykan-Aminosäure-Komplex herzustellen;
Nachweisen des markierten O-Glykan-Aminosäure-Komplexes; und
Charakterisieren einer Glykosylierung des Proteins und/oder des Peptids mittels LC-, MS- oder LC/MS-Analyse durch den Nachweis des markierten O-Glykan-Aminosäure-Komplexes.

2. Verfahren nach Anspruch 1, wobei der Verdauungspuffer MS-kompatibel ist.

3. Verfahren nach Anspruch 1, wobei die Protease allein oder in Kombination mit einem anderen Typ Proteasen vermischt wird.

4. Verfahren nach Anspruch 3, wobei die Proteasen sequentiell mit dem Verdauungspuffer vermischt werden.

5. Verfahren nach Anspruch 1, wobei das Protein teilweise verdaut wird.

6. Verfahren nach Anspruch 1, wobei das Verfahren ferner den Schritt des Reinigens des Glykan-Aminosäure-Komplexes mit HILIC-SPE und des Isolierens des Glykan-Aminosäure-Komplexes aus der Matrix umfasst, oder wobei das Verfahren ferner den Schritt des Reinigens des markierten Glykan-Aminosäure-Komplexes und des Isolierens des markierten Glykan-Aminosäure-Komplexes umfasst.

## Revendications

1. Procédé de caractérisation de la glycosylation d'une protéine ou d'un peptide comprenant les étapes consistant à :
fournir une pluralité de protéines et/ou peptides O-glycosylés ;
mélanger la pluralité de protéines et/ou peptides O-glycosylés avec une pluralité de protéases et un tampon de digestion, la pluralité de protéases clivant la protéine et/ou le peptide avec la sérine, la thréonine, la tyrosine ou l'hydroxylysine dans un processus de digestion protéolytique en solution pour produire un complexe de O-glycane et d'acides aminés ;
marquer le complexe de O-glycane et d'acides aminés avec un réactif de marquage pour produire un complexe de O-glycane et d'acides aminés marqué ;
détecter le complexe de O-glycane et d'acides aminés marqué ; et
caractériser la glycosylation de la protéine et/ou du peptide par une analyse CL, SM ou CL/SM et par la détection du complexe de O-glycane et d'acides aminés marqué.

2. Procédé selon la revendication 1, dans lequel le tampon de digestion est compatible SM.

3. Procédé selon la revendication 1, dans lequel la protéase est mélangée seule ou en combinaison avec un autre type de protéases.

4. Procédé selon la revendication 3, dans lequel les protéases sont mélangées séquentiellement avec le tampon de digestion.

5. Procédé selon la revendication 1, dans lequel la protéine est partiellement digérée.

6. Procédé selon la revendication 1, le procédé comprenant en outre l'étape consistant à purifier le complexe de glycane et d'acides aminés par HILIC SPE et à isoler le complexe de glycane et d'acides aminés de la matrice, ou le procédé comprenant en outre l'étape consistant à purifier le complexe de glycane et d'acides aminés marqué et à isoler le complexe de glycane et d'acides aminés marqué.
